Europäisches Patentamt

**European Patent Office**

**Office européen des brevets**

(11) Publication number: **0 156 176**
**A1**

(12) ## EUROPEAN PATENT APPLICATION

(21) Application number: **85102180.8**

(22) Date of filing: **27.02.85**

(51) Int. Cl.⁴: **C 12 M 1/36**
**G 06 F 15/46**

(30) Priority: **28.02.84 US 584463**

(43) Date of publication of application:
**02.10.85 Bulletin 85/40**

(84) Designated Contracting States:
**AT BE CH DE FR GB IT LI LU NL SE**

(71) Applicant: **CETUS CORPORATION**
**1400 Fifty-Third Street**
**Emeryville California 94608(US)**

(72) Inventor: **Zeitlin, James Andrew**
**33 Keswick Court**
**Oakland California 94611(US)**

(72) Inventor: **Merrill, Roy Dewitt**
**51 Estabueno**
**Orinda California 94563(US)**

(74) Representative: **Vossius Vossius Tauchner Heunemann Rauh**
**Siebertstrasse 4 P.O. Box 86 07 67**
**D-8000 München 86(DE)**

(54) **Fermentation control system.**

(57) A unitary control system controls a plurality of individual digital control systems (10; 11; 12; 13) for fermenter units operating independently of each other in response to a multiplicity of measured variables representing the reaction rates and end products of each fermenter unit. The energy input quantities to each unit are controlled independently either locally or from a master control unit (20) to achieve optimum fermentation conditions. Each of the plurality of fermentation systems, running under widely varying reaction conditions, is operated to produce any selected microorganism under substantially differing, or substantially identical process reaction conditions. The individual fermenter control system (10; 11; 12; 13) includes an executive program using a high speed microprocessor bus (44) for operating data storage, program storage and signal processing and an application program using a low speed input and output bus (42) for continuously monitoring and transmitting measured condition data and end effector control data on a sequential basis. Operator intervention for modifying program values or end effector operation without system upset is provided to both the local, microprocessor computer and the central, minicomputer controller.

./...

FIG._1.

Case: 2111
Cetus Corporation
Emeryville, California 94608, USA

# FERMENTATION CONTROL SYSTEM

This invention relates to control of fermentation systems. More particularly, it relates to systems for selectively controlling a plurality of fermenters from a master control unit, or controlling each fermenter unit locally to regulate individually and selectively a multiplicity of modules or tasks for each operating variable of such fermention systems.

It is an object of the invention to provide a unitary control system to operate a plurality of fermenter units independently of each other in response to a multiplicity of measured variables representing the reaction rates and end products of each fermenter unit. The energy input quantities, both chemical and physical, to each unit are controlled independently either locally or from a master control unit to achieve optimum fermentation conditions. Accordingly each of the plurality of fermentation systems, operating under widely varying reaction conditions, may be operated to produce any selected microorganism under substantially differing, or substantially identical process reaction conditions. Such a plurality of fermentation processes may also be entirely unrelated to each other, but still are controlled either from the central control unit or at each local control unit.

The fermentation control system of the present invention is particularly useful in developing fermentation conditions for research and development of recombinant microorganisms, such as DNA or RNA, or the like, to improve and optimize yield in a manufacturing process, or for full commercial production of such micro-organic species on a batch basis.

In developing commercial fermentation processes it is frequently necessary to operate a plurality of batch fermentation systems simultaneously to optimize various reaction conditions for reproduction of microorganisms, including bacteria, protozoans, yeast, algae and cells. In such simultaneously operated fermenters, the rate of addition of energy to the fermentation culture or "broth" includes both chemical and physical inputs. Chemical energy is supplied by oxygen or air to maintain dissolved oxygen at a proper level; addition of acid or base optimizes the pH of the fermenting broth; antifoam agents are added to control foam; and where not initially sufficient, nutrients, such as dextrose or sugar, are added. Physical energy variables that are controlled in a fermentation process include mechanical stirring or agitation of the mixture to improve oxygen solution and maintain uniformity of growth conditions throughout the culture; temperature of the broth is controlled by heat input or removal. Other measured and controlled variables suitable for analysis and control of the progress of the fermentation process, include sampling fermentation off-gases, primarily oxygen and carbon dioxide, and chemical or biological examination of the growing microorgansism and their waste or by-products in the growth media.

Heretofore in fermentation control systems it has been common to use individual loop controllers for each input variable. These generally take the form of electrical analog circuits in which an electrical signal either current or voltage is generated by a transducer measuring a variable parameter, or condition, such as temperature, dissolved oxygen, or the like. Then, in accordance with any departure from such measured variable from a desired value, an end effector, or final control element, such as a valve is selectively driven in an amount and to an extent to return the measure variable to the set value. Such loop controllers, also require means to generate an electrical feedback signal to indicate the actual amount and extent of the corrective movement of the input device, or effector. Two measurements are used to prevent undue oscillation of the measured variable about a selected value, these are, (1) the setpoint which is normally a range of acceptable values (control band) above and below the setpoint, and (2) the effector drive signal, which is derived from the difference between the setpoint and the measured condition of the variable being controlled. The derived signal is a proportional, integral and derivative function of such difference. Where multiple variables are involved as in a fermentation system, each loop controller must be separately modified to achieve a desired end result. Process controllers for each individual variable are well known in the art, such as temperature controllers to measure the temperature of the culture and then control the heat or cooling to maintain the set temperature. Normally such loop controllers are regulated independently and operate to maintain the input energy or quantity (heat), so that the measured variable (temperature) is within the control band. However in fermentation systems, biological growth processes are substantially unpredictable and seldom linear. Correspondingly the demand for physical and chemical inputs to maintain optimum growth conditions vary widely and frequently at differing rates over different periods of time. Further modification of any one input may modify the need for other inputs. Additionally, the control modes for different end effectors are quite dissimilar; for example, the amount of correction applied to one end effector in response to either the absolute change or the rate of change in the measured variable from a setpoint or length of time the measured variable has been away from the setpoint are closely related to interaction of other end effectors. For these reasons, it is highly desirable to provide an arrangement for interrupting or intervening in, the operation of each control module or task either from a central control system in response to centrally measured conditions such as power loss or off-standard product or locally in response to one or more locally measured values including upset, alarm or error conditions to permit any control variable to be modified as the fermentation process proceeds.

Proposals have been made to provide overall central control of a plurality of process variables by analyzing resulting off-gas, by-products or products of the process. Such analyses may include chromatographic examination of the off-gas, or mass spectrometer systems to detect and measure the concentration of various gases in the off-gas stream being generated by microorganisms being reproduced by fermentation. It has also been proposed to use such measurements to regulate the setpoint and control band of such individual process variables. In accordance with such known loop controllers these controllers typically provided for proportional, rate and reset functions, using proportional - integral - derivatives or on-off control algorithms. Such algorithms permit manual adjustment of the setpoint and control band in each control loop to attempt to achieve desired reaction rates in the fermentation process. Such control loops are most suitable for certain control tasks where the measured variable and the input quantity are directly related. However, in control of fermentation processes used for commercial development of biologically engineered microorganisms, generally each measured variable depends upon a plurality of interrelated input quantities. For example, dissolved oxygen in the culture is primarily dependent upon the rate oxygen is taken up by the reproducing organisms and the rate of oxygen addition to the culture in the form of dissolved oxygen (DO). DO addition is dependent upon the rate of flow of either oxygen gas or air and the rate at which such supplied oxygen goes into solution. The latter is in turn dependent upon agitation of the culture liquid and the temperature of the liquid. Further, each of such variables is rate and reset.

-4-

dependent and affected by "upset" imposed by abrupt changes in growth rate or by external manipulation of inputs, either manual or automatic.

In commercial fermentation involving the growth of genetically engineered microorganisms, cultures, it is highly desirable to be able to operate the same or similar processes simultaneously in a number of fermentation vessels, yet control independently the fermentation process conditions in each vessel. Where the number of individual fermentation vessels may range from say 10 to 30, provision of individual loop controllers for each of the numerous variables and individual computing systems for each set of multiple variables becomes prohibitively expensive in equipment and operator maintenance.

For the foregoing reasons, it is a particular object of the present invention to provide a fermentation control system in which a minicomputer or other central computer is capable of direct digital control of the operation of a plurality of fermentation vessels each including an independently operable local direct digital controller operating a plurality of control modules or tasks to regulate the process variables either singly or jointly in each fermentation vessel. Such plurality of fermentation processes under supervision of the master control, or minicomputer, may be similar to, or wholly dissimilar from, each other. In each individual fermentation process a plurality of control task modules are established in an Erasable, Programmable Read Only Memory (EPROM) unit and/or a Random Access Memory (RAM) unit to control a local computer in the form of a microprocessor circuit. Such microprocessor circuit selectively controls on a heirarchical basis, each task module measuring circuits to determine separate process variables, including but not limited to nutrient addition, pH, foam level, temperature, agitation, air and oxygen. Similarly, the microprocessor, interconnected to each task module operates one or more of a plurality of control effectors, or input control elements to modify the quantity of energy supplied to each system. In accordance with the present invention, such end effector task modules are controlled by one or more measuring devices to modify the input quantity in an amount and to an extent to maintain singly or jointly each measured process variable within a predetermined set of limits or range of values. The measured quantities and control variables are operated through a multiplex bus which on a sequential basis converts each measured signal from analog to digital values and as required converts digital input values to the control units to the required power signals whether continuous or semi-continuous A.C., D.C., or modulated pulses

In each fermentation vessel, a single microcomputer operates on a time-share basis to control all task modules, including recording, data display, alarms, operator controlled keyboard and master control intervention. Further, in controlling the primary tasks of continuously and automatically regulating the multiple end effectors in response to input signals from the respective measuring circuits, the system provides for manual over-ride for the end effectors without introducing discontinuous control signals from a feedback control algorithm. Accordingly, the plurality of energy inputs to the fermentation system, either physical or chemical, are supplied in an amount and to an extent to maintain a setpoint for each measured variable in response to any desired combination of energy inputs, whether generated automatically in a pre-programmed sequence from the ROM and EPROM or manually generated by operator intervention. Such intervention may be generated either locally or at a master controller, such as a minicomputer. Setting of values for the setpoints and control band, as well as overall interaction of a plurality of variables to control operation of the fermentation system, is preferably through a single central minicomputer which may manually or automatically adjust such setpoints and control bands during any portion of the fermentation process. Additionally, the central computer may regulate the process in response to manual input from an operator keyboard. This acts to provide manual override of the preset values. Such override without process upset is accomplished with direct digital control because there is no feedback signal from movement of the end effector. The local, programmable microcomputer then controls each control task module for each process variable during operation of the fermenter vessel. In accordance with the present invention the central computer not only may establish initial setpoints and control band values but also generates new values for either or both in response to measured product or output values from the individual fermentation vessels. Preferably such measured product values are used continuously to update inputs for calculation by algorithms to generate new control limits for the end effector signals. Such signals are transmitted by a common bus to regulate the energy inputs for the individual vessel controller. Accordingly there is provided to the master controller updated information on a demand basis, for each task module under local control at each individual fermentation vessel. At the same time, the individual fermentation units are sequentially and continuously controlled locally on a real-time basis. The present invention thereby provides economy of control units, including investment in equipment to measure and control the multiple inputs affecting production of an end product. At the same time such control may be applied through sophisticated programs based on algorithms, either developed on the basis of the end product or selectively developed during progress of the fermentation run. Such programs may be selectively used in any of the individual vessel control systems without modification of the control circuits of the microcomputer for each individual fermentation vessel.

Further, the unitary control system provided by the present invention permits a multiplicity of individual fermentation processes to be monitored and controlled from a single central control unit, or the process may be completely controlled by the local control unit.

Further objects and advantages of the present invention will become apparent from the following detailed description of the preferred embodiments of the invention taken with the accompanying drawings which form an integral part of the present specification.

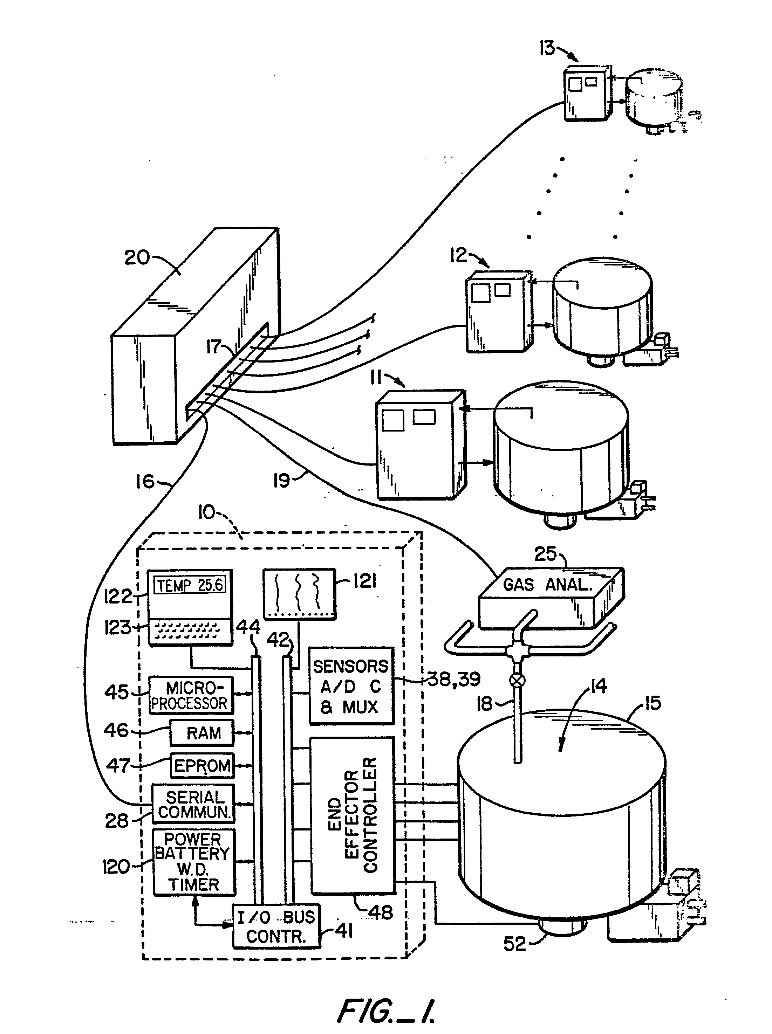

Fig. 1 is a overall view of a fermentation system indicating a single master station control for a plurality of fermentation vessels, each with its associated controls and measuring systems and the interconnection from the vessel to a programmable local microprocessor for operating each individual control loop of the fermenter vessel. The diagram also indicates supervisory or heirarchic control by the central process system to detect, record and update set values of each of the input variables and measured output quantities or to activate alarms at each of the plurality of fermentation control units.

Fig. 2 is a schematic diagram of a single fermenter vessel shown in Fig. 1 indicating the measured variables and the end effectors controlling the physical and chemical inputs to the fermentation system.

Fig. 3 is a block diagram indicating generally the organization of the bus arrangements of the present invention for a local microprocessor, memory and intervention units to program an input/output bus system to measure condition variables and transmit power signals to end effectors with display and recording for the fermentation system illustrated in Fig 2.

Fig. 4 is a block diagram indicating the division of local and central computer tasks for the system of Fig. 1.

Fig. 5 is a block diagram indicating schematically the division of functions in the control system of the present invention between the Executive Program and Application Program and Data and Memory Arrays Modules.

Fig. 6 is a block diagram indicating in greater detail the Application Program Task, which are selectively or sequentially controlled by the System or Executive Program and the Hardware inputs and outputs supervised by the local microcomputer and memories for execution of the Application Tasks.

Referring now to the drawings and particularly to Fig. 1, there is shown an overall schematic representation of the fermentation control system embodying the present invention. A plurality of individual or batch fermentation process control systems are indicated generally as 10, 11, 12 and 13. Each batch fermentation vessel, such as 15, is operated under the control of a local direct digital controller such as 10. Controller 10 alone provides a plurality of measuring circuits and effector control circuits, each of which locally controls one or more of a multiplicity of interrelated input variables to grow selected microorganisms by fermentation in a culture broth 14 in vessel 15. Master control 20 provides central direct digital control allowing intervention in any selected task for each of such multiplicity of measured input and controlled output variables at each of the individual batch fermentation process controllers 10, 11, 12, 13.

In general, controller 20 may be a minicomputer arranged to operate through a communication link, such as bus 17 or line 16 to a Serial Communications Interface 28 controlled by Serial Communications Task 124 to initially set limits for each control task supervised by local controller 10. This includes each individual variable to be measured and each output controlled to operate an end effector to modulate the energy supplied to the fermenter such as agitation rate, oxygen, air supply, etc. carried out in each fermentation vessel 15.

As further indicated schematically, off-products of vessel 15 may be sampled by various forms of sophisticated end-product analyzing systems. For example, line 18 may introduce off-gas from vessel 15 into a multistream gas chromatograph 25. Although not shown, samples of liquid or broth 14 may be analyzed, as by a mass spectrometer or other liquid or solids monitoring systems. As discussed below the purpose of such product analysis equipment is to provide process alarms or to assist in developing control algorithms to modify control band limits through the master controller in accordance with widely diverse operating conditions in the fementation process as it proceeds. Such operating conditions of course range from a highly dilute or dispersed initial state for microorganisms in culture 14 to high density conditions at the end of each run. In development of optimum fermentation systems it is highly desirable for the operator to be able to monitor and intervene in any selected control task to maintain maximum growth of the desired microorganism under such significantly different ambient conditions.

In accordance with the present invention, it is possible automatically or manually to establish set points or control ranges for each individual controller, through display and keyboard units at the master controller and individual lines, such as 16, between each fermentation controller and the master controller. Line 16, or bus 17, may also selectively control other inputs to each fermenter system from the master controller. For example, sets of initial conditions for each control variable may be assigned to each individual controller either through programs loaded automatically into a RAM or the EPROM unit. Such input conditions may also be manually modified by operator control through a keyboard and alpha/numeric display unit (not shown) at central unit 20 or locally through keyboard 123 and display 122. These units may also provide manual override of the end effectors. Desirably, data is continuously displayed, and recorded on a selected basis, as by strip chart recorder 121. Data may also be logged for the entire process run in each individual fermenter.

It will be understood of course that the individual fermentation controllers and the fermenters themselves are designed to optimize reproduction of microorganisms of all types either under similar conditions or widely different conditions for the same microorganisms. Alternatively the controllers may operate the fermentation systems for reproduction of microorganisms of very dissimilar characteristics. As used herein "microorganisms" is intended to comprise bacteria, protozoa, yeast, viruses and algae, including slime molds and cells including helio cells or neoplasms.

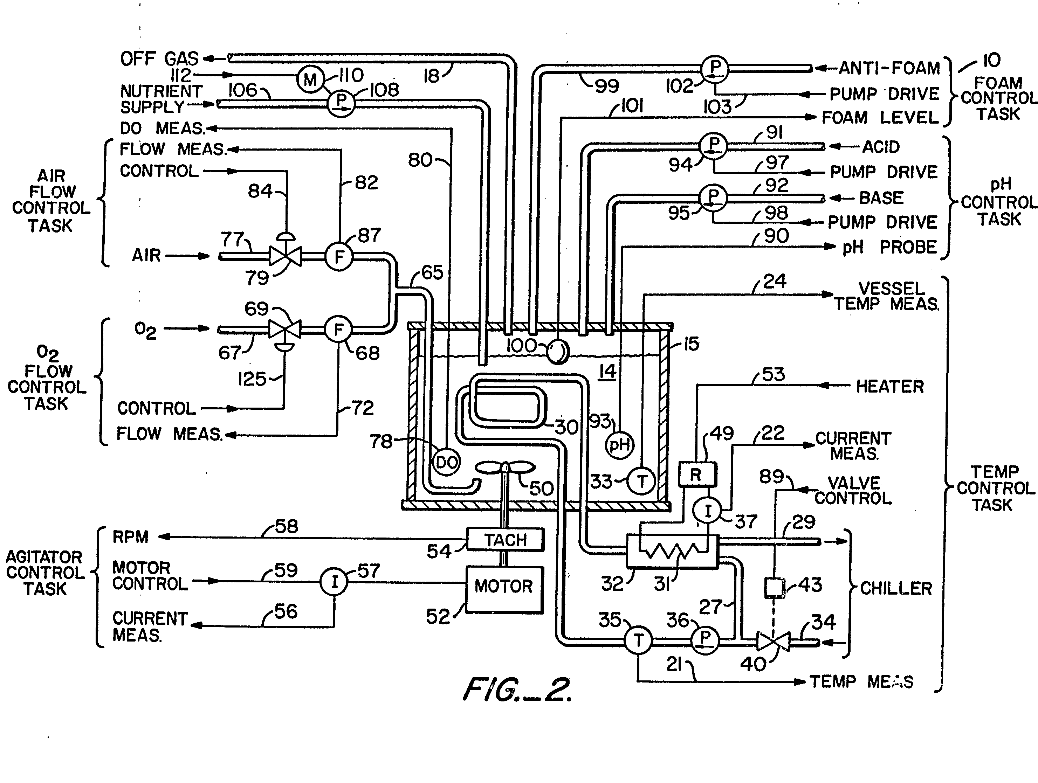

Fig. 2 represents one of the fermentation vessels 15 and illustrates the essential end effector and sensor tasks operating through control system 10 to control each individual energy input to the fermentation vessel and to measure the resultant changes of state of culture 14, as the desired microorganisms grow.

-9-

In a typical fermentation vessel control system the energy inputs to promote growth of desired microorganisms in the broth or culture are: nutrient, (e.g. glucose), mechanical stirring or agitation, oxygen, air, chemicals for foam control, pH control chemicals (acid or base), and heat, added or subtracted. Each of such energy input control systems includes means for measuring at least one condition affected by such energy input whether applied singly or jointly. For simplicity in explaining the operation of the present invention the significant control variables including measured values and end effector controls for energy inputs are illustrated in Fig. 2. In this embodient these are indicated as the six control tasks which require hardware implementation to measure and control input quantities; these are air flow, oxygen flow, agitator speed, foam, pH and temperature. Since these quantities are basic to all fermentation processes, they are the most readily measured. However, other values, such as dissolved oxygen, nutrient input and off-gas are of vital importance in controlling fermentation. Their relation to the control tasks as performed by the present invention will be discussed in greater detail below.

As indicated in Fig. 2, the temperature system includes measuring the temperature of culture 14 by resistive thermodetector (RTD) 33. Because the fermentation process is frequently exothermic, cooling water from line 34 is supplied through control valve 40 to heat exchanger coil 30 in tank 15. The cooling water may also be heated by auxiliary heater, electrical coil 31, to maintain a given temperature. Such water is recirculated from heat exchanger 32 to coil 30 by pump 36 through line 27. The temperature of cooling water circulated to tank heat exchanger coil 30, is measured by another RTD 35, and current flow through electrical heater 31 in heat exchanger 32 is measured by ammeter 37. Water passing through heat exchanger 32 either flows out through return line 29 or is recirculated by pump 36.

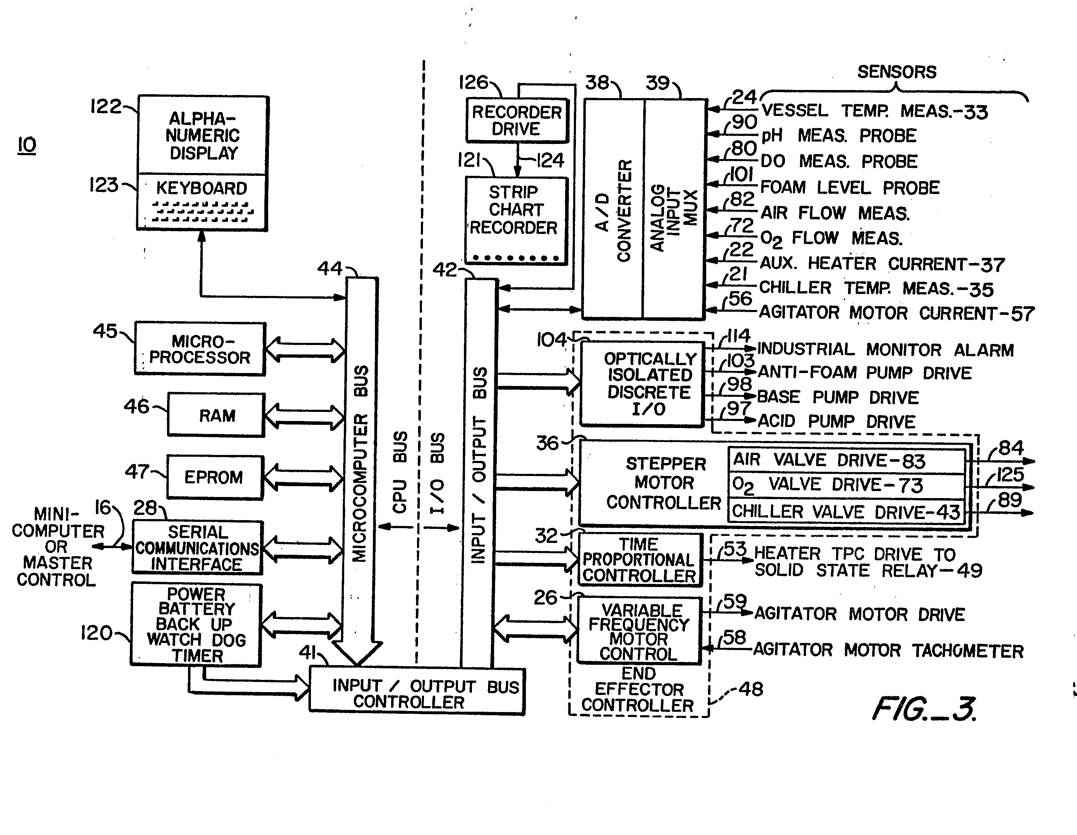

In conventional control systems each detected electrical signal representative of the temperature, such as that at RTD 35 is used in an electrical feedback circuit to modify directly the end effector, such as a valve 40 in cooling water line 34 to return the temperature to a set value. Similarly current to heater 31 and RTD 33 would be arranged in a similar control loop for direct control of heater 31. However, as particularly distinguished from such an arrangement of loop controllers, in the present invention the electrical signals from temperature and current sensing elements 33, 35 and 37, as indicated in Fig. 3, are each continuously and periodically sampled through analog input/multiplex unit (MUX) 39 and transmitted by analog to digital converter (ADC) 38 over input/output (I/O) bus 42 through bus controller 41 to microcomputer 45 over high speed microcomputer bus 44. Such input values are compared with setpoint and range values stored either in random access memory (RAM) 46 or in the erasable programmable read-only memory (EPROM) 47, both also connected to bus 44. Such values may, of course, be either fixed, time-varied or pre-programmed. Control signals representing the extent of variations of the measured values from the setpoint are processed by microcomputer 45 and correcting signals are in turn transmitted over input/output bus 42 through bus controller 41 to a plurality of end effector controllers, indicated generally as 48.

In the temperature control task 23 such effectors are chiller valve drive 43 which preferably actuates a step motor drive to open or close valve 40 in line 34 in discrete steps and solid state relay 49 which regulates current input to heater 31. Chiller valve drive 43 is actuated through stepper motor control 36 (Fig. 3) to supply electrical pulses. Heater 31 is supplied with power through time proportional controller 32 and solid state relay 49. Controller 32 varies the average power applied to resistance heater 31.

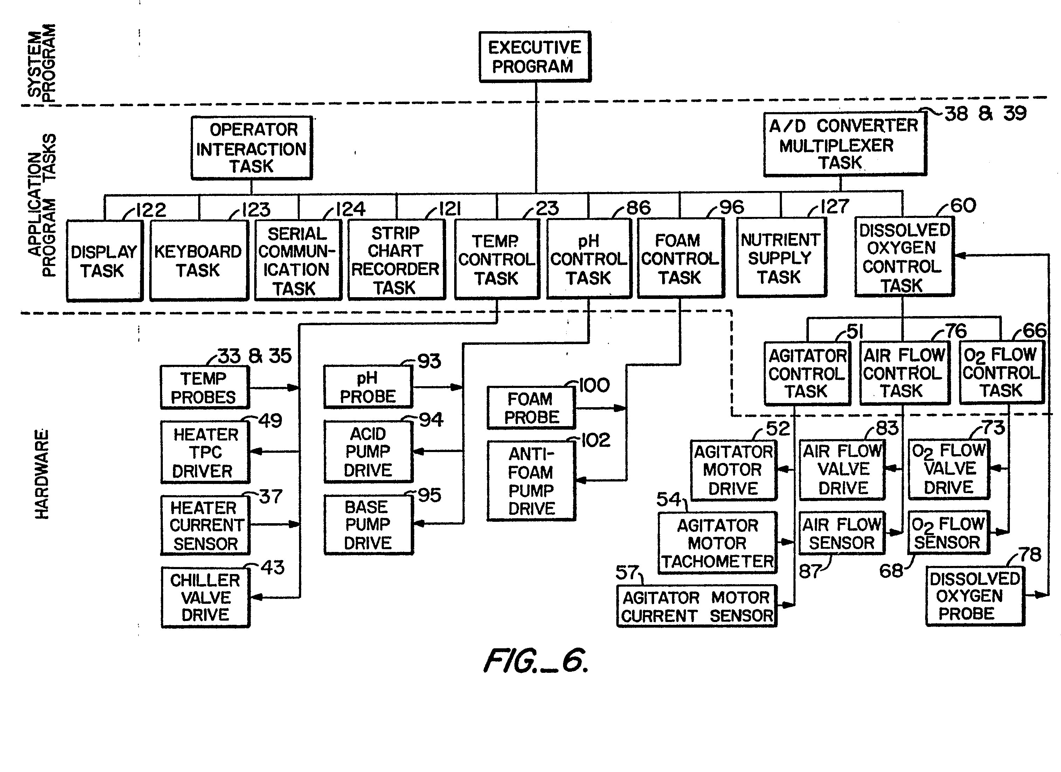

As indicated in Figs. 1 and 3, microprocessor bus 44 provides a highspeed (less than 10 microseconds) communication link between the stored data, and programs in EPROM 47, and RAM 46 with local microcomputer 45. These three units together embody the Executive Program as indicated in Figs. 5 and 6 for regulating each of the multiple application program tasks to control and monitor each fermenter system. Functionally the read-only memory RAM 46 stores each variable that is measured and the status of each end effector drive unit. It also stores the operator selectable limit values for the individual measured variables for display and actuation of indicators when the limits of such values are exceeded, including alarms or other indicators. The control programs including algorithms for calcuation of restorative signals to each of the end effectors, based upon the displacement of the measured value from the preset value are stored in the programmable memory unit EPROM 47.

From the foregoing it will be seen that the fermenter control includes a two-level hierarchical system in which a plurality of micro-processor based local controllers, such as 10 embody all control functions that require sequential periodic, high duty cycle, measurements and real-time responses to correct system energy inputs. Each local controller is therefore capable of operating fully independent of the central computer. Thus, the minicomputer central unit 20 is able to handle less frequent but more sophisticated functions. These may include data logging and periodic off-gas or product measurements for supervisory control of the individual fermentators. Such supervisory control programs may be individually tailored for a given fermentation process. The output data from such programs, executing on minicomputer 20, may control any individual or a selected plurality of local microcomputer, fermenter control systems 10, 11, 12 and 13. This is through communication line 16 and serial communicator 28 of an individual local controller, such as 10. In this way central station control 20 and local microprocessor control 10 may transfer operational data, control parameters, including range and setpoints, to manually set or adjust values and if desired, to set up on-line optimization routines.

As will be unoerstood by those skilled in the fermentation art, the rate of growth of microorganisms may be detected by their uptake of oxygen which varies widely from beginning to end of a batch run. In accordance with the present invention such wide variations are accomodated by close control of the temperature to within less than 0.1 degree Centrigrade. Temperature control system mooule or task 23, as above described provides such a local control and is essential to maintain a more direct control for growth which is provided by the Dissolved Oxygen (DO) Control System or Task 60. As indicated in Fig. 6, DO Task 60 controls Air Flow Control System or Task 76, Oxygen Flow Control System or Task 66 and Agitator Control System or Task 51 in response to Dissolved Oxygen Probe 78, located in tank 15.

In accordance with the present invention, the method of maintaining the dissolved oxygen content of culture 14 is preferably by using each of the control systems in cascade. Namely, the stir rate first increases to maintain full dispersion of available oxygen throughout tank 14 through Agitator Control System 51. As indicated in Fig. 2, agitator or stirrer 50 is driven by motor 52; the energy input is detected by tachometer 54 and ammeter 57, respectively measuring agitator speed and current flow to motor 52. As indicated, tachometer 54 operates through variable frequency motor control 26, and current flow is detected through Analog Input MUX 39. Both quantities are used to modulate power to A.C. motor 52, also through control 26.

Upon agitation reaching a given, or desired maximum rate, the dissolved oxygen control operates through Air Flow Control Task 76 and Air Flow Control Valve 79, to begin or increase the mass flow rate of air through line 65 into broth 14. As indicated, a local control loop in air flow control task 76 measures such flow through anomometer 87. The anomometer signal may be compared with flow from the air supply (not shown) through line 77, under control of valve 79. Regulation of the opening of valve 79, also step-motor driven, is in response to digital signals received from control line 84. The measured value for air flow is transmitted as an electrical signal over line 82. In a preferred form, flow detector 87 is in the form of an anomometer, which includes an internal hot wire resistor in a bridge circuit (not shown) to generate the desired electrical signal.

To maintain the total amount of oxygen available in the culture, as detected by dissolved oxygen probe 78 and transmitted over line 80 to analog MUX unit 39 and AD unit 38, the flow rate of air is increased until a given or maximum value is obtained with given or maximum agitation. At that point, flow of oxygen under control of $O_2$ Flow Control System or Task 66 begins or increases.

The oxygen flow control system is substantially similar to the air flow control system. As indicated, oxygen flows from a source (not shown) through line 67 under control of valve 69 and anemometer 68 to line 65. Oxygen flow through anemometer 68 is measured and the signal is transmitted by line 72 to signal converters 38 and 39. It also provides a feed back signal to $O_2$ Valve Drive 73 through control line 125 for valve 69 which also is preferably step motor driven by Step Motor Controller 36 (Figure 3).

Further, in accordance with the control method of the present invention, with maximum agitation and maximum air flow, dissolved oxygen in culture 14 is monitored by DO probe 78 until oxygen flow is increased to a predetermined value or maximum. At that point, the air supply is reduced to common supply line 65 so that oxygen partial pressure of the culture medium may attain a higher value. In summary, the dissolved oxygen system algorithm operates as microbe metabolism increases first to increase stir rate to maximum, then increases air sparging to a maximum flow, then increases oxygen flow to a maximum and then decreases air flow to increase oxygen partial pressure in tank 15 to maintain a desired DO content as the rate of growth increases.

The pH Control System, or Task, 86, regulates supply of acid or base from supply lines 91 or 92, respectively, in response to measured values transmitted from pH detector 93 in culture 14 by line 90 to ADC unit 38 and MUX unit 39. Pumps 94 and 95 control acid and base addition, respectively. As indicated in Fig. 2 the power for operating pumps 94 and 95 is supplied through lines 97 and 98, respectively, from Optically Isolated Discrete I/O unit 104.

Similarly, Anti-Foam Control System or task 96 includes a foam level probe or sensor 100 at the upper end of vessel 15 connected by line 101 to signal input units 38 and 39. Anti-foam solution is controlled by pump 102 from supply line 99. Power for pump 102 is supplied through line 103 by Optically Isolated Discrete I/O unit 104.

The Nutrient Supply Task 127 generally provides nutrient to culture 14 in the form of a dextrose solution. It is supplied through line 106 and pump 108 to tank 15. Desirably pump 108 is driven by motor 110 through power line 112 either continuously at variable speeds as through Variable Frequency Motor Control 26 or intermittently through Optically Isolated Discrete I/O unit 104. In general, the nutrient flow setpoint is controlled as a function of both pH and DO.

Since each of the energy supply units is controlled by direct digital control by microcomputer unit 45, the setpoint, and the range of values above and below the setpoint defining the control band, as well as the other control algorithm coefficients, may each be selectively fixed over a wide range of values by Keyboard Task and Display Task units, as exemplified by local keyboard unit 123 and alpha-numeric display unit 122. Alternatively they may be fixed by master controller 20 operating through local Serial Communications Task unit 28. In a specific case, for example, the DO and pH controls employ feedback algorithms using control bands and proportionality constants so adjusted in setting up each fermentation run. Such values are stored in EPROM 47 and are compared by microcomputer 45 with stored digital signals in RAM 46 generated by any form of analog signal converted to digital form or any direct digital signal produced by one of the measuring probes or devices. Correspondingly corrective digital signals actuate power bus 42 through controller 41 to transmit needed power signals to operate the end effector units 29, 32, 36 and 104 to operate the valves, pumps, motors, heaters, etc. to produce the required energy inputs by digital or analog movement of any electrical, hydraulic or pneumatic final control elements.

From the foregoing description it will be understood that the present invention provides optimum use of sophisticated and expensive measuring and control devices, particularly ADC unit 38 and analog/MUX unit 39 and the power conversion provided by the actuator units of End Effector Controller 48. By heirarchical control of the microcomputer units 10 in each fermenter system by minicomputer 20, it is possible to use inexpensive computer units, such as an Intel 8080, to operate each local direct digital controller. Thus complex signals involving comparison of input and power signals, dependent upon fixed or progressively changing computer algorithms may be generated, programmed and/or stored for operation of any of a multiplicity of system parameters or variables in any of a plurality of local fermenter systems. Such algorithms may be applied either jointly or wholly independently of each other to any fermenter. This is important in fermentation systems because the growth of microorganisms is frequently unpredictable and generally non-linear during different portions of the growth cycle. Accordingly, by the present invention the degree of complexity of each individual control system may be altered without interfering with other control parameters in the same fermenter or other fermenters.

From the foregoing it will be apparent that in computer implemented automatic digital control of a fermentation system, multiple physical and chemical inputs and unpredictable growth of microorganisms present exceedingly complex problems of both condition measurement and end effector controls. As particularly distinguished from chemical processes or simple material handling processes where only a few elements need be controlled to optimize operation of a system, fermentation systems require continuous control of certain conditions, such as temperature, dissolved oxygen and agitation, but only periodic control of such conditions as foam, pH, or nutrient addition.

In accordance with the present invention the problem of continuous monitoring and control of the temperature agitation and heat tasks, as well as the intermittent tasks, are sequentially sampled and powered on a continuously repeated basis by microprocessor 45. Such information is transmitted to and from microcomputer via input/output bus 42 as above described. Bus 42 is a relatively slow bus operating on the order of one signal pulse per 1 to 100 milliseconds. With such pulse intervals the entire set of tasks are monitored in from about one to five seconds and corrective signals generated. However, in accordance with the Executive Program, the detected signals from each task are processed and corrective signals generated only when a programmed or external event occurs or a specific time interval elapses. Upon occurrence of such an event or time interval, the executive program executes the appropriate application task until the next such event or time interval occurs. In this way each task is controlled by the microcomputer with direct digital control.

To assure that failure of power to the microcomputer does not interrupt operation of the continuously monitored tasks, e.g. temperature, agitation and dissolved oxygen, a battery back-up with suitable watchdog timer and alarm systems, as indicated by block 120, are arranged to supply power to microprocessor control system 10, if conventional power is lost. Additionally, the heirarchic control arrangement for each fermenter system by control computer 20 and local control 10 may be such that regular sequential signals are fed back to unit 20 by local unit 10. Failure of such signals to occur may be used to indicate failure of unit 10 to central unit 20. To provide a fail-safe arrangement in the local controller, Watch Dog Timer shuts off power to the heaters, agitator and stepper motors if the Executive Program does not generate a reset pulse every 50 milliseconds. This unit also provides Power Battery Back-up to insure retention of setpoints, control coefficients and other parameters in the local controller in the event of power failure. It also activates an Industrial Monitor Alarm through line 114 and control unit 104.

For local monitoring, a Strip Chart Recorder Task is through unit 121 which has its input desirably interconnected to Input/Output Bus 42. The strip chart is driven through Recorder Drive 126 and line 124.

In accordance with the present invention intervention by actuation of display and keyboard tasks by units 122 and 123 is accorded priority in the programming of sequential tasks controlled by the Executive Program operating through microcomputer 45. Similarly, the Serial Communications task is given priority by the Executive Program over all other tasks.

-16-

To effect proper transfer between operator inserted values and end effectors each inserted value must be within preset limits established before each run. Accordingly, each inserted value is first compared with such preset range of values stored in RAM 46 before end effector pulses are generated by microprocessor 45 and transmitted by I/O Bus Controller 41 to power bus 42.

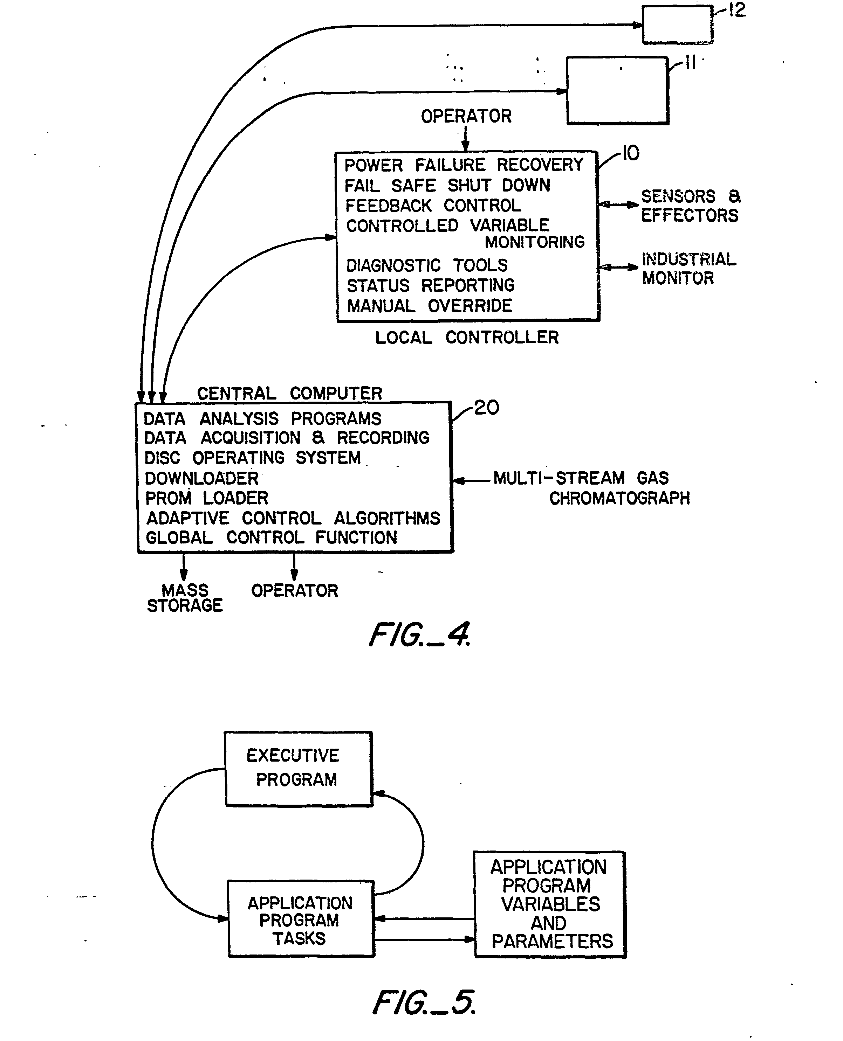

Fig. 4 indicates the general forms of control that can be exercised with the minicomputer or central control system 20 and the local or microcomputer controlled system 10. Fig. 5 is a schematic diagram of the system logic and implementation of such logic, as more fully shown in Fig. 6.

While only a few examples of the invention have been disclosed in detail, various changes will become apparent to those skilled in the art of automatic fermentation systems from the described embodiments. All modifications or changes coming within the spirit and scope of the claims are intended to be covered thereby.

CLAIMS:

1. A fermentation control system characterized by:

a plurality of individual batch fermentation process direct digital controllers,

each controller including a plurality of measuring circuits for determining a plurality of fermentation process operating conditions, each measuring circuit having at least one condition detector, a plurality of end effector circuits for controlling each energy input required in variable amounts to continue operation of said fermentation process, means for converting the signals from each of said plurality of measuring circuits to digital pulses, means for generating a digital output signal for each of said plurality of said end effector circuits in response to signals from at least one of said condition detectors dependent upon the energy input associated with the corresponding end effector, means for transmitting the digital pulses representative of said energy input from each of said measuring circuits and the digital output signal to each of said end effector circuits on a time multiplexed basis over a single input-output bus, the signal transmitted over said single input-output bus being time sequentially controlled by a microprocessor bus including microprocessor data storage means, said microprocessor means being arranged to receive said digital measuring pulses and to generate said digital output pulses in response to programmable means stored in said storage means, and generating a power control input for each of said end effectors in an amount and to an extent to maintain continuously the operating condition of said end effector at a known value in response to the last digital output signal received by said microprocessor means; and

a master control unit operatively connectable to said microprocessor bus of each of said plurality of direct digital controllers, said master control unit being operative to transmit program data to initialize and update conditions in said storage means or for receiving and transmitting digital pulses for direct digital control of any end effector of any of said batch fermentation controllers.

2. A fermentation process control system wherein the reaction processes are carried out in a plurality of batch fermentation vessels which are operated simultaneously under differing physical interactions and/or metabolic steps and each of said vessels includes a plurality of reaction condition measuring systems and a plurality of energy input end effectors which are controllable in response to said measuring systems characterized by:

a plurality of separate direct digital process control circuits for regulating each of a plurality of end effectors to maintain each variable energy input of each fermentation process in each of said plurality of vessels within a predetermined range of values,

each of said circuits including means for detecting analog or digital signals representative of at least one measured value and analog or digital means for controlling power signals to an end effector to supply input energy to one of said vessels in an amount and to an extent to maintain said measured value within its predetermined range, and means for sequentially transmitting said signals to and from each of said circuits over a common input/output bus to a microprocessor over a microprocessor bus, said microprocessor controlling said end effectors to maintain said measured signals within said range of values by transmitting digital signals to an end effector power controller adapted to generate a power signal having an amplitude, phase and frequency for operating a selected end effector drive means in response to at least one of the detected signals associated therewith; and

a common minicomputer for independently controlling the range of measured values for actuation of said power controller digital signals in each of said plurality of fermentation vessels including means for establishing a setpoint within said range of values and the limits of said range above and below said setpoint.

3. A fermentation process control system according to claim 2 characterized in that said power signals are transmitted over said common input/output bus from a power source through a signal converter to condition each signal to the required electrical signal for the drive means to operating each of said end effectors.

4. A method of controlling a plurality of fermentation processes from a central control station for optimum operation of each of said processes under rapidly changing metabolic conditions and differing energy input interactions characterized by the steps of:

generating at said central station a digital control program including time variable setpoints and control band values for a selected fermentation process in each of a plurality of fermenter vessels, each vessel including a primary local control system for a plurality of energy inputs to maintain operation of said selected fermentation process, transmitting and storing said digital control program in a local direct digital process controller including a master clock for sequentially and continuously comparing a plurality of time variable setpoints and control band values with a corresponding plurality of measured process variables,

repetitively and periodically sampling a plurality of measured process variables in said fermentation process, comparing said measured variables with said stored setpoint and control band values, periodically and repetitively transmitting control signals to an end effector power controller in response to deviations of each said measured value from its time varying stored value, and in response to said control signals varying the input power to said end effectors in an amount and to an extent to maintain said measured value at said time varying stored value, and

periodically modifying said setpoint and control band values in said central station in accordance with changes in the growth rate of microorganisms being reproduced in said fermentation process, and transmitting said modified values to update said local direct digital process control program in accordance with said growth rate.

5. A batch culture fermentation process for growth of microorganisms wherein the population density of said microorganisms in a culture solution grows at varying rates, said rates being dependent upon a plurality of growth conditions in said solution including temperature, pH, dissolved oxygen, carbon dioxide evolution, and culture agitation rates characterized by the steps of:

sequentially and repetitively detecting at least one electrical quantity representative of the magnitude of each of said growth conditions in said culture solution,

synchronously and sequentially comparing a digital value of each detected electrical quantity over a range of digital values above and below one representing a preselected time value of a setpoint for said growth condition,

in response to a difference in said detected preselected digital value and said time value of said setpoint generating a plurality of digital signals corresponding to the direction and extent of said difference, and sequentially generating a power signal for at least one end effector controlling an input quantity into said culture solution in accordance with said plurality of digital difference signals for each of said measured growth conditions,

said power signals being sequentially generated at a rate substantially slower than the rate for repetitively detecting said electrical quantities, and applying each of said power signals to a selected one of said effectors to return said growth condition toward said preselected time value of said setpoint.

6. A fermentation control process for automatically maintaining the growth of microorganisms in a batch culture medium with increasing population of such microorganisms by maintaining the dissolved oxygen content of the culture medium at a predetermined level characterized by the steps of continuously detecting the dissolved oxygen content of said culture medium and in response thereto increasing the agitation rate of said media to a predetermined maximum, then increasing the flow of air into said medium to a predetermined maximum flow rate, then increasing oxygen flow into said medium to a predetermined maximum flow rate and then decreasing the air flow rate to a lower predetermined value to increase the partial pressure of oxygen of said medium and maintaining said conditions to completion of fermentation in said batch culture.

7. A fermentation control process wherein the reaction process is carried out in a batch fermentation vessel which includes a plurality of reaction condition measuring systems and a plurality of energy input end effectors which are controllable in response to growth of micro-organisms in said vessel characterized by:

a plurality of separate direct digital process control circuits for regulating each of said plurality of end effectors to maintain each variable energy input to said fermentation vessels within a predetermined range of values,

each of said circuits including means for detecting and transmitting and electrical signal in digital form representative of the measured value of at least one reaction condition and digital signal generating and transmitting means for controlling power signals to an end effector to supply an input energy to said vessel in an amount and to an extent to maintain said measured value within its predetermined range, and means for sequentially transmitting said detected and generated signals to and from each of said circuits over a common input/output bus and bus controller means to microprocessor means over a microprocessor bus, said microprocessor means including digital signal storage means, digital signal generating means process program storage means, said microprocessor means controlling said end effectors in accordance with differences between said measured digital signals and the range of digital values stored in said program storage means for transmitting said generated digital signals to an end effector power controller, bus controller means being adapted to generate a power signal having an amplitude, phase and frequency for operating a selected end effector drive means in response to at least one of the detected digital signals associated therewith; and

means for independently controlling the range of stored values in said microprocessor means for generation of said power controller digital signals for each of said plurality of end effectors including means for establishing a setpoint within said range of values and the limits of said range above and below said setpoint during operation of the fermentation process.

8. A fermentation control process according to claim 7 characterized in that said independent control means directly modifies said generated power signals which are transmitted over said common input/output bus from a power source through a signal converter to condition each signal to the required electrical signal for the drive means to operating each of said end effectors.

FIG._I.

*FIG._2.*

FIG._3.

0156176

POWER FAILURE RECOVERY
FAIL SAFE SHUT DOWN
FEEDBACK CONTROL
CONTROLLED VARIABLE
                MONITORING

DIAGNOSTIC TOOLS
STATUS REPORTING
MANUAL OVERRIDE

LOCAL CONTROLLER

OPERATOR

SENSORS &
EFFECTORS

INDUSTRIAL
MONITOR

CENTRAL COMPUTER

DATA ANALYSIS PROGRAMS
DATA ACQUISITION & RECORDING
DISC OPERATING SYSTEM
DOWNLOADER
PROM LOADER
ADAPTIVE CONTROL ALGORITHMS
GLOBAL CONTROL FUNCTION

MULTI-STREAM GAS
CHROMATOGRAPH

MASS
STORAGE

OPERATOR

*FIG._4.*

EXECUTIVE
PROGRAM

APPLICATION
PROGRAM
TASKS

APPLICATION
PROGRAM
VARIABLES
AND
PARAMETERS

*FIG._5.*

FIG._6.

## DOCUMENTS CONSIDERED TO BE RELEVANT

| Category | Citation of document with indication. where appropriate, of relevant passages | Relevant to claim | CLASSIFICATION OF THE APPLICATION (Int Cl 4) |
|---|---|---|---|
| A | GB-A-2 094 024 (NEW BRUNSWICK SCIENTIFIC)<br>* Page 1, lines 69-85; page 1, lines 115 - page 2, line 6 * | 1,4 | C 12 M 1/36<br>G 06 F 15/46 |
| A | US-A-3 926 737 (WILSON)<br>* Column 1, line 36 - column 2, line 4 * | 1,4 | |
| A | BIOTECH 83, 4th-6th May 1983, pages 295-306, London, GB;<br>TOMOAKI SUMITANI et al.:<br>"Automatic control of fermentor using microcomputer"<br>* Page 295, line 1 - page 298, line 8 * | 1,4 | |
| A | US-A-4 001 557 (STEPHENSON)<br>* The whole document * | 1,4 | TECHNICAL FIELDS SEARCHED (Int. Cl 4)<br><br>G 06 F<br>G 05 B<br>G 05 D<br>C 12 M |

The present search report has been drawn up for all claims

| Place of search | Date of completion of the search | Examiner |
|---|---|---|
| THE HAGUE | 03-05-1985 | BARRACO G.S. |